# EUROPÄISCHE PATENTSCHRIFT

(11) **EP 3 362 144 B1**
(45) Veröffentlichungstag und Bekanntmachung des Hinweises auf die Patenterteilung: **17.07.2019**
(21) Anmeldenummer: 16790902.7
(22) Anmeldetag: 17.10.2016
(51) Int. Cl.: A61N 1/39, A61G 12/00

(54) **MODULARES INTENSIVTHERAPIEGERÄT**
MODULAR INTENSIVE THERAPY APPLIANCE
UNITÉ DE SOINS INTENSIFS MODULAIRE

(30) Priorität: 15.10.2015 DE 102015117581
(43) Veröffentlichungstag der Anmeldung: 22.08.2018
(73) Patentinhaber: Kagan, Eugen, 53859 Niederkassel Lülsdorf (DE)
(72) Erfinder: Kagan, Eugen, 53859 Niederkassel Lülsdorf (DE)
(74) Vertreter: Wagner Albiger & Partner Patentanwälte mbB
(86) Internationale Anmeldenummer: PCT/EP2016/074859
(87) Internationale Veröffentlichungsnummer: WO 2017/064318

(56) Entgegenhaltungen:
- DE-A1-102012 103 029
- US-A1- 2013 304 142
- US-B1- 6 352 504

## Beschreibung

Die Erfindung betrifft ein modulares Intensivtherapiegerät, umfassend ein Basisgerät mit mindestens einem in einer Andockfläche ausgebildeten Modulsteckplatz sowie mindestens ein Funktionsmodul, welches an dem mindestens einen Modulsteckplatz des Basisgeräts andockbar und lösbar verriegelbar und elektrisch mit diesem verbindbar ist, wobei jeder Modulsteckplatz und jedes Funktionsmodul mit zueinander komplementär ausgebildeten lösbaren Verriegelungseinrichtungen und elektrischen Verbindungseinrichtungen ausgebildet sind.

Modulare Intensivtherapiegeräte im Sinne der Erfindung werden auch als Critical Care Units (CCU) bezeichnet und beschrieben, wozu beispielsweise auf die DE 10 2012 103 029, US 6,352,504 B1 und US 2013/0304142 A1 verwiesen wird.

Die modularen CCU's dienen dazu, eine individuelle Versorgung eines Patienten flexibel herzustellen, indem das Basisgerät in Form einer Docking Station, die die Ansteuerung und Energieversorgung aller angedockten Module übernimmt, mit den benötigten Funktionen, wie Vitalparametermonitoring, Defibrillator, Beatmungsgerät und dergleichen mehr ausgerüstet wird, wobei entsprechende Funktionsmodule, die die gewünschten Funktionen mit sich bringen, an den Modulsteckplätzen des Basisgeräts befestigt werden bzw. zwischen verschiedenen Basismodulen ausgetauscht werden. Das Basismodul übernimmt überdies auch die Datenanzeige, die Datenverarbeitung sowie die Archivierung und Übertragung an Zentraldatenstationen, wie CIS im Krankenhaus.

Darüber hinaus kann ein solches modulares Gerät im Falle des Defekts eines einzelnen Funktionsmoduls durch Austausch leicht wiederhergestellt werden.

Nachdem derartige vorkonfigurierte CCU's zunächst lediglich für den stationären Einsatz geeignet waren, da sie entsprechend großen Platzbedarf und keine funktionale Flexibilität aufwiesen, sind in jüngster Zeit verstärkt modulare Intensivtherapiegeräte vorgeschlagen worden, die sich aufgrund ihrer kompakten Abmessungen und auch aufgrund einer integrierten mobilen Stromversorgung durch Akkus auch für den außerstationären Einsatz beispielsweise im Rettungsdienst eignen. Dies hat insbesondere den Vorteil, dass ein bereits vom Rettungsdienst an das modulare Intensivtherapiegerät angeschlossener Patient beim Eintreffen in einer Klinik und Aufnahme in der Intensivstation nicht nochmals neu an entsprechende Therapiegeräte angeschlossen werden muss, sondern das bereits angeschlossene modulare Intensivtherapiegerät den Patienten begleitend auch in die Intensivstation mitgeführt und dort in den stationären Betrieb übernommen werden kann. Dabei werden je nach Anwendung keine Geräte ausgetauscht, sondern lediglich entsprechende für den jeweiligen Patienten benötigte Funktionsmodule angeschlossen und verwendet.

Bei den bislang bekannten modularen CCU's wird bei besonders kompakten Bauformen, wie beispielsweise gemäß der bereits erwähnten DE 10 2012 103 029 A1 eine Anordnung bevorzugt, bei der die Vorderseite des Basisgeräts nahezu vollständig von einem berührungssensitiven Bildschirm eingenommen wird, der sowohl den Status der einzelnen Therapien sowie den Patientenmonitor darstellt als auch die notwendigen Bedienereingaben zur Steuerung des Intensivtherapiegeräts ermöglicht. Entsprechend werden die Funktionsmodule an entsprechenden Modulsteckplätzen auf der Rückseite im Bereich einer Andockfläche platziert, indem die Funktionsmodule seitlich, d.h. im Wesentlichen parallel zur Andockfläche z.B. in Aufnahmeschienen eingeschoben werden. Zwar bietet eine derartige Anbringung hohe Stabilität und Rüttelfestigkeit für den mobilen Betrieb, bringt jedoch den Nachteil mit sich, dass das An- und Abdocken der Funktionsmodule entsprechend großen Platzbedarf neben der Andockfläche erfordert, der beispielsweise das Handling in einem Rettungswagen oder auf der Intensivstation verschlechtert. Überdies hatte diese Anbringung auch zur Folge, dass die an den einzelnen Funktionsmodulen vorhandenen Anschlussfelder zum Beispiel für den Anschluss von Sondenkabeln, Beatmungsschläuchen und dergleichen mehr an einer definierten Seite des Intensivtherapiegerätes angeordnet sind. Dies führt zu Einschränkungen hinsichtlich der Handhabung und Anordnung insbesondere auf der Intensivstation, da die Anschlussfelder möglichst zum Patienten zeigen sollten, um die zu verlegenden Leitungen und Schläuche zum Patienten möglichst kurz zu halten. Dies erfordert mitunter aufwändige Umbauten, wenn ein Patient in der Intensivstation eintrifft.

Aufgabe der vorliegenden Erfindung ist es daher, ein modulares Intensivtherapiegerät der eingangs genannten Art in besonders kompakter Bauweise vorzuschlagen, welches sich flexibel an die örtlichen Gegebenheiten anpassen lässt, höchste Verbindungsfestigkeiten sowohl in mechanischer also elektrischer Hinsicht und geringsten Platzbedarf aufweist.

Zur Lösung der gestellten Aufgabe wird erfindungsgemäß ein modulares Intensivtherapiegerät mit den Merkmalen des Patentanspruchs 1 vorgeschlagen.

Vorteilhafte Ausgestaltungen und Weiterbildungen der Erfindung sind Gegenstand der abhängigen Ansprüche.

Die Erfindung ist in den beigefügten Ansprüchen definiert. Hier offenbarte Aspekte, Ausführungsformen und Beispiele, die nicht in den Umfang der beigefügten Ansprüche fallen, sind nicht Teil der Erfindung und werden lediglich zu Veranschaulichungszwecken bereitgestellt.

Der erfindungsgemäße Vorschlag sieht vor, dass das die Verriegelungseinrichtungen und die elektrischen Verbindungseinrichtungen im Bereich der Andockfläche ausgebildet und so angeordnet sind, dass das mindestens eine Funktionsmodul senkrecht zur Andockfläche an dem mindestens einen Modulsteckplatz andockbar und lösbar verriegelbar ist.

Durch diese erfindungsgemäße Anordnung senkrecht zur Andockfläche wird der Platzbedarf beim Anbringen bzw. Abnehmen der einzelnen Funktionsmodule am bzw. vom Basisgerät minimiert, da die Funktionsmodule im Wesentlichen senkrecht auf die Andockfläche aufgesetzt bzw. senkrecht von dieser abgehoben werden können. Die Verriegelung und elektrische Kontaktierung erfolgt auf einer Ebene, nämlich im Bereich der Andockfläche, und zwar senkrecht zum Basisgerät.

Die Verriegelungseinrichtungen werden nach einem Vorschlag der Erfindung von an den Funktionsmodulen vorstehenden Laschen mit diese durchsetzenden Bohrungen und andererseits von zugehörigen Einsteckschlitzen und in die Bohrungen lösbar eingreifenden Verriegelungsbolzen im Bereich jedes Modulsteckplatzes des Basisgeräts gebildet. Werden diese Verriegelungseinrichtungen symmetrisch zur Mittelachse angeordnet, ermöglichen sie die gewünschte um 180° zueinander versetzte Orientierung der Funktionsmodule und deren Befestigung in den zwei unterschiedlichen Orientierungen am Basisgerät.

Nach einem weiteren Vorschlag der Erfindung sind die Verriegelungsbolzen federbelastet im Basisgerät gehaltert und greifen beim Einstecken der Laschen in die Einsteckschlitze insoweit selbsttätig in die Bohrungen ein, um die Laschen am Basisgerät zu verriegeln. Mittels zugeordneter Antriebe, die auf die Verriegelungsbolzen einwirken, kann über am Basisgerät angeordnete Freigabeorgane ein teilweiser Herauszug der Verriegelungsbolzen aus den Bohrungen bewirkt werden, wobei als Freigabeorgane beispielsweise elektrische Taster, mechanische Hebel oder mechanische Tasten am Basisgerät vorgesehen sind. Weiterhin ist bevorzugt, wenn jeder Modulsteckplatz mit einem zugeordneten Freigabeorgan versehen ist.

Mit Vorteil werden die Verriegelungsbolzen bei Betätigung der Freigabeorgane mittels der zugeordneten Antriebe nur soweit aus den Bohrungen herausgezogen, dass diese mit ihrem freien Ende noch innerhalb der Bohrungen der Laschen verbleiben, so dass die Laschen lediglich unter Aufbringung einer vorbestimmten Zugkraft aus den Einsteckschlitzen herausziehbar sind, in denen dann die Verriegelungsbolzen gegen die Federlast aus den Bohrungen herausgleiten. Dadurch wird sichergestellt, dass beim Betätigen der Freigabeorgane das entsprechende Funktionsmodul nicht selbsttätig vom Modulsteckplatz des Basisgerätes abfällt, sondern nach Betätigen des Freigabeorgans unter Aufwendung einer entsprechenden Zugkraft und insoweit nur durch Hinzutun eines Bedieners vom Basisgerät getrennt und abgenommen werden kann.

Weiterhin können die Freigabeorgane einen Sensor zur Erfassung einer Betätigung des jeweiligen Freigabeorgans aufweisen und es ist eine mit dem Sensor verbundene Steuerungseinrichtung vorgesehen, mittels derer bei vom Sensor erfasster Betätigung des Freigabeorgans die Antriebe zeitverzögert aktivierbar sind, um die Verriegelungsbolzen aus den Bohrungen herauszuziehen. Durch diese Zeitverzögerung ist es z.B. möglich, zunächst die Software eines Funktionsmoduls herunterzufahren, bevor die Verriegelungsbolzen aktiviert werden und insoweit das Abnehmen des Funktionsmoduls unter Trennung der elektrischen Verbindungseinrichtungen bewirkt werden kann. Darüber hinaus kann die Steuerungseinrichtung außerdem mit einem Sensor zur Erfassung der in die Einsteckschlitze eingesteckten Laschen verbunden sein, so dass bei nicht erfolgtem Herausziehen der Laschen binnen eines vorgebbaren Zeitintervalls die Antriebe von der Steuerungseinrichtung deaktivierbar sind und die Verriegelungsbolzen erneut die Laschen vollständig verriegeln. Hierdurch wird eine hohe Sicherheit gegen unbeabsichtigtes Lösen der Funktionsmodule vom Basisgerät bei versehentlicher Betätigung des Freigabeorgans erreicht, da sich die Funktionsmodule nach Ablauf des vorgebbaren Zeitintervalls selbsttätig wieder mit dem Basisgerät verriegeln.

Nach einem alternativen Vorschlag der Erfindung sind die Verriegelungseinrichtungen von an den Funktionsmodulen vorstehenden Haken mit zugehörigen Einstecköffnungen in der Andockfläche und in die einstecken Öffnungen lösbar eingreifenden und mit den Haken in Wirkverbindung tretenden Verriegelungsscheiben als Freigabeorgane im Bereich jedes Modulsteckplatzes des Basisgeräts gebildet. Die Anordnung vorstehender Haken an den Funktionsmodulen beeinträchtigt den im Innern der Funktionsmodule zur Verfügung stehenden Bauraum für die Aufnahme der erforderlichen Funktionsbauteile nicht und gewährleistet eine besonders stabile mechanische Verbindung zum Basisgerät.

Alternativ kann auch vorgesehen sein, dass die Verriegelungseinrichtungen von an der Andockfläche im Bereich jedes Modulsteckplatzes des Basisgeräts vorstehenden Haken und schwenkbaren Verriegelungshebeln als Freigabeorgane gebildet sind, die mit den zugehörigen Einstecköffnungen an den Funktionsmodulen in Wirkverbindung bringbar sind.

Nach einem weiteren Vorschlag der Erfindung sind die Verriegelungseinrichtungen und die elektrischen Verbindungseinrichtungen des mindestens einen Funktionsmoduls in zwei um 180° zueinander versetzten Orientierungen an dem mindestens einen Modulsteckplatz verriegelbar und elektrisch mit diesem verbindbar sind. Hierdurch wird es möglich, je nach räumlicher Gegebenheit die Funktionsmodule so an den Modulsteckplätzen des Basisgerätes zu befestigen, dass die seitlich angeordneten Anschlussfelder der Funktionsmodule wahlweise zur rechten oder linken Seite des Intensivtherapiegerätes weisen. Erfindungsgemäß haben die im Rahmen des modularen Intensivtherapiegerätes gemäß der Erfindung verwendbaren Funktionsmodule somit keine Vorzugsrichtung mehr, sondern sind um 180° wendbar an den jeweiligen Modulsteckplätzen befestigbar.

Nach einem Vorschlag der Erfindung wird vorgeschlagen, dass die Verriegelungseinrichtungen und die elektrischen Verbindungseinrichtungen symmetrisch zu einer durch den Modulsteckplatz verlaufenden Mittelachse angeordnet sind.

Weiterhin kann vorgesehen sein, Funktionsmodule auszubilden, welche mehrere Modulsteckplätze belegend an dem Basisgerät lösbar verriegelbar und elektrisch mit diesem verbindbar sind, so dass mehr Bauraum innerhalb des Gehäuses des Funktionsmoduls für die Integration entsprechender funktionaler Gerätschaften zur Verfügung steht. Beispielsweise können die Funktionsmodule nicht nur einen Modulsteckplatz belegen, sondern auch zwei, drei oder vier derartige Modulsteckplätze je nach Anforderung.

Die elektrischen Verbindungseinrichtungen der Funktionsmodule und der Modulsteckplätze sind nach einem weiteren Vorschlag der Erfindung von Kontaktplatten gebildet, die jeweils eine Vielzahl von Kontakten aufweisen, die in einer um 180° versetzten Anordnung dupliziert sind und bei an einem Modulsteckplatz verriegeltem Funktionsmodul aneinander zur Anlage kommen. Durch die duplizierte Anordnung ist es dabei unerheblich, in welcher der beiden Anordnungen das Funktionsmodul am Modulsteckplatz verriegelt wird und es wird gleichermaßen die vollständige Kontaktierung der Kontaktplatten und der darin aufgenommenen Vielzahl von Kontakten sichergestellt.

Zur Schaffung einer ausreichenden Rüttelfestigkeit, wie sie insbesondere beim Mobileinsatz z.B. in einem Rettungswagen oder Rettungshubschrauber gefordert ist, vorgeschlagen, dass die Kontakte mindestens einer Kontaktplatte der elektrischen Verbindungseinrichtung federbelastet in Richtung der jeweils anderen Kontaktplatte angeordnet sind, um einen ausreichenden Anpressdruck der beiden Kontaktplatten zueinander zu gewährleisten.

Hierbei kann sowohl die gesamte Kontaktplatte federnd bzw. schwimmend im Basisgerät und/oder dem Funktionsmodul gelagert sein oder aber die einzelnen Kontakte mindestens einer Kontaktplatte weisen eine derartige Federbelastung in Richtung der jeweils anderen Kontaktplatte auf.

Weitere Ausgestaltungen und Einzelheiten des erfindungsgemäßen modularen Intensivtherapiegerätes werden nachfolgend anhand von Ausführungsbeispielen in der Zeichnung näher erläutert. Es zeigen:
- Figur 1: in einer perspektivischen Darstellung die Ansicht auf ein modulares Intensivtherapiegerät gemäß der Erfindung;
- Figur 2: eine weitere Ansicht auf das modulare Intensivtherapiegerät gemäß Figur 1;
- Figur 3: die Rückansicht des modularen Intensivtherapiegeräts gemäß der Erfindung;
- Figur 4: in perspektivischer Darstellung ein Funktionsmodul gemäß der Erfindung;
- Figur 5: die Ansicht des Funktionsmoduls gemäß Figur 4;
- Figur 6: die Aufsicht auf das Funktionsmodul gemäß Figur 4 in am Basisgerät verriegelter Position;
- Figur 7: in perspektivischer Darstellung eine weitere Ausgestaltung eines erfindungsgemäßen Funktionsmoduls;
- Figur 8: eine Ansicht des Funktionsmoduls gemäß Figur 7;
- Figur 9: in perspektivischer Darstellung das modulare Intensivtherapiegerät gemäß der Erfindung mit einem Funktionsmodul gemäß Figuren 7 und 8;
- Figur 10: eine weitere Ansicht des modularen Intensivtherapiegeräts gemäß Figur 9;
- Figur 11: die Vorderansicht des modularen Intensivtherapiegeräts in perspektivischer Darstellung.
- Figur 12: eine weitere Ausführungsform der Erfindung in einer Ansicht gemäß Figur 1
- Figur 13: eine weitere Ausführungsform der Erfindung in einer Ansicht gemäß Figur 1
- Figur 14: die Seitenansicht der Ausführungsform gemäß Figur 13
- Figur 15: eine weitere Ausführungsform der Erfindung in eine Ansicht gemäß Figur 1
- Figur 16: die Aufsicht auf ein Funktionsmodul gemäß Figur 15

Aus den Figuren ist ein modulares Intensivtherapiegerät ersichtlich, welches ein mit 1 bezeichnetes Basisgerät umfasst, dessen Vorderseite 10 nahezu vollständig von einem berührungssensitiven Bildschirm ausgebildet wird, der zur Steuerung und Anzeige der Funktionen des modularen Intensivtherapiegerätes dient.

Die Unterseite des Basisgeräts 1 wird von einem rechtwinklig vom Vorderteil 10 abstehenden Bodenteil 13 gebildet, welches einerseits die Standfläche ausbildet und andererseits Bauraum für die Aufnahme von Komponenten des Basisgeräts 1, beispielsweise einer Datenverarbeitungseinheit und/oder einer Stromversorgung aufnimmt. In der Seitenansicht bilden die Vorderseite 10 und das Bodenteil 13 eine etwa L-förmige Konfiguration.

Oberseitig ist darüber hinaus ein Tragegriff 14 angeformt, der es gestattet, das modulare Intensivtherapiegerät per Hand zu transportieren.

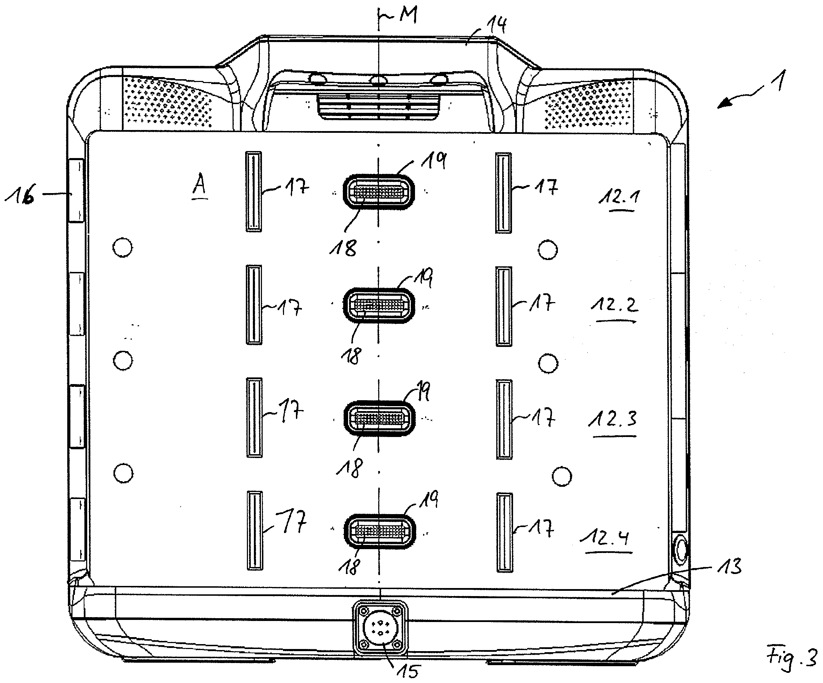

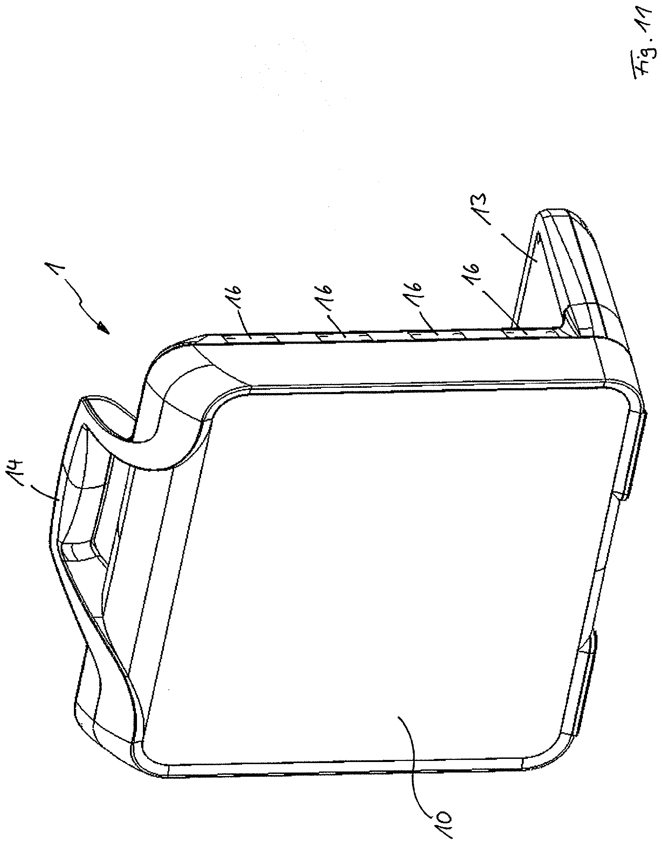

Das aus den Figuren 3 und 11 ersichtliche Basisgerät 1 umfasst nur geringe bis keine intensivtherapeutische Funktionalität, um einen Patienten zu überwachen bzw. zu therapieren. Dies wird durch nachfolgend näher erläuterte Funktionsmodule 2 ermöglicht, die entsprechend der gewünschten Anforderung an auf der Rückseite der mit Display ausgerüsteten Vorderseite 10 angeordneten Modulsteckplätzen 12.1, 12.2, 12.3 und 12.4 angedockt und mit der Datenverarbeitungs- und Stromversorgungseinheit des Basisgeräts 1 in Verbindung gebracht werden können. Wie aus der Figur 3 ersichtlich, sind die hier vorgesehenen vier Modulsteckplätze 12.1 bis 12.4 übereinstimmend dimensioniert und übereinander angeordnet, jedoch sind auch hiervon abweichende Anordnungen und Anzahlen der Modulsteckplätze ohne weiteres möglich.

Die Modulsteckplätze 12.1,12.2,12.3 und 12.4 sind übereinander im Bereich der Rückseite 11 des Basisgeräts 1 angeordnet, wobei die Rückseite 11 zugleich die ebene Andockfläche A ausbildet, an der die Funktionsmodule 2 angedockt und befestigt werden.

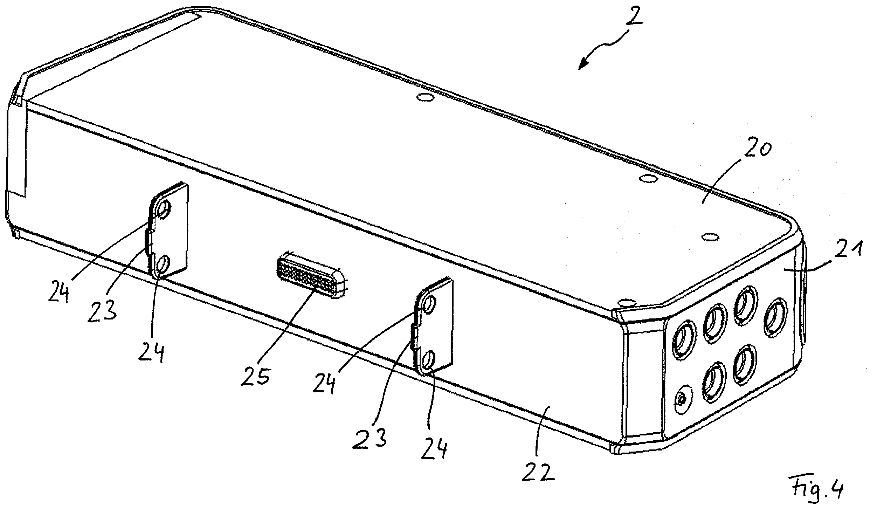

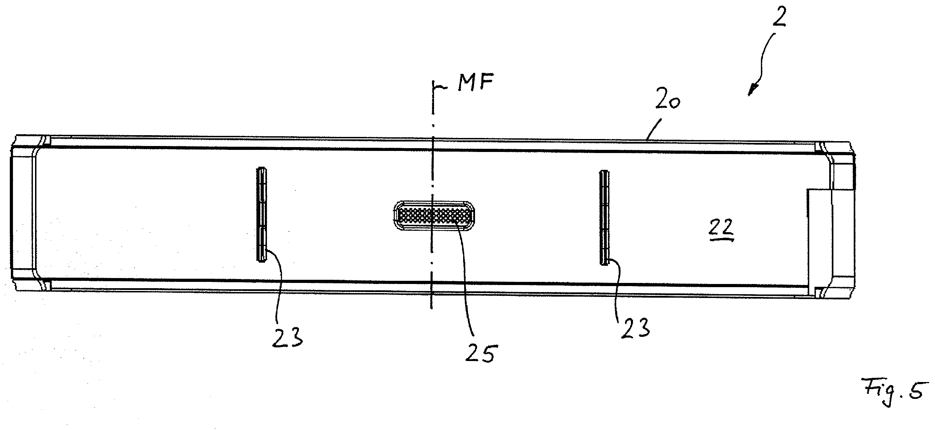

Ein einzelnes Funktionsmodul, welches mit Bezugszeichen 2 bezeichnet ist, ist aus den Figuren 4 und 5 ersichtlich und umfasst ein etwa quaderförmiges Gehäuse 20, wobei an einer Seite des Gehäuses 20, die auch als Andockseite 22 bezeichnet wird und dem Anschluss an das Basisgerät 1 dient, eine über die Andockseite 22 vorstehende Kontaktplatte 25 als Teil von elektrischen Verbindungseinrichtungen zum Basisgerät 1 sowie zwei in gleichem Abstand zur Kontaktplatte 25 angeordnete und beispielsweise aus Stahlblech gefertigte vorstehende Laschen 23 mit darin ausgebildeten und übereinander angeordneten Bohrungen 24 als Teil der Verriegelungseinrichtungen zum Basisgerät 1 vorgesehen sind.

Benachbart zur Andockseite 22 ist an einer Schmalseite des quaderförmigen Gehäuses 20 ein Anschlussfeld 21 vorgesehen, so dass ein solches Modul gemäß Darstellung der Figur 4 über in das Anschlussfeld 21 und in dort vorhandenen Buchsen eingesteckten Verbindungskabel bzw. -schläuche (nicht dargestellt) mit einem Patienten verbunden werden kann. Das in der Figur 4 dargestellte Funktionsmodul 2 kann beispielsweise ein EKG-Modul sein, ist jedoch auf diese Funktionalität nicht beschränkt.

Um das aus den Figuren 3 und 11 ersichtliche Basisgerät 1 insoweit mit der Funktionalität des aus den Figuren 4 und 5 ersichtlichen Funktionsmoduls 2 auszurüsten, wird das Funktionsmodul 2 an einem der auf der Rückseite des Basisgeräts 1 vorhandenen Modulsteckplätze 12.1, 12.2, 12.3, 12.4 befestigt bzw. angedockt, wobei die Auswahl des Modulsteckplatzes beliebig ist.

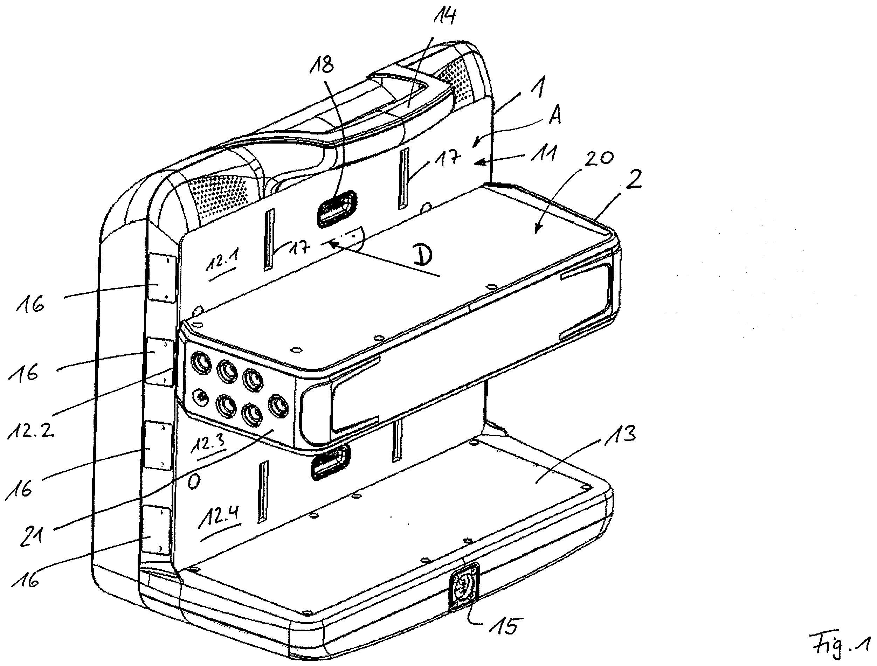

In der Darstellung gemäß Figur 1 ist diese Befestigung beispielsweise am Modulsteckplatz 12.2 vorgenommen worden.

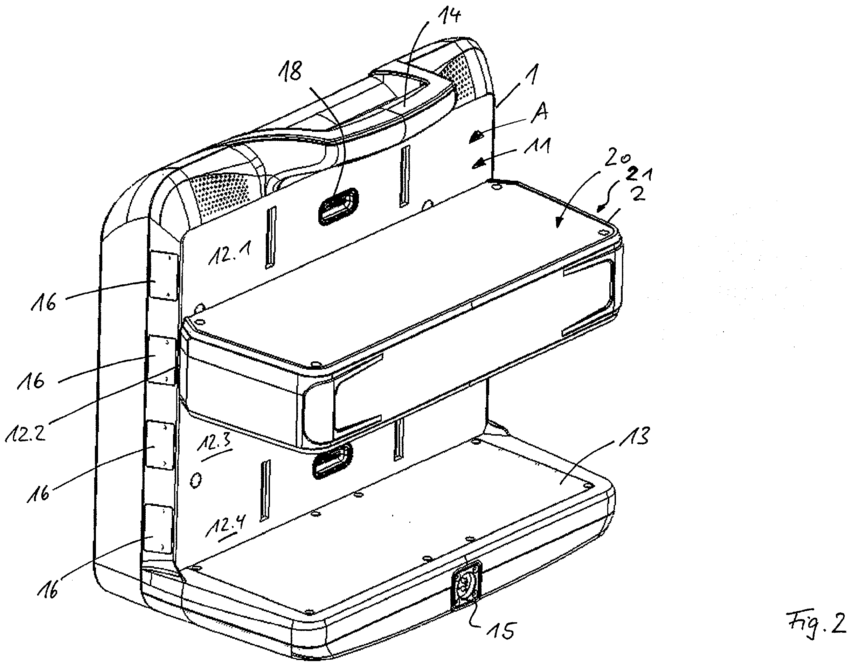

Alle Modulsteckplätze 12.1 bis 12.4 weisen, wie aus der Darstellung gemäß Figur 3 ersichtlich, elektrische Verbindungseinrichtungen in Form von komplementär zur vorstehenden Kontaktplatte 25 des Funktionsmoduls 2 ausgebildeten Kontaktplatten 18 auf, ferner als lösbare Verriegelungseinrichtungen komplementär zu den am Funktionsmodul 2 vorstehenden Laschen 23 ausgebildete Einsteckschlitze 17. Insoweit ist es möglich, wie in den Figuren 1 und 2 dargestellt, das Funktionsmodul mit seinen vorstehenden Laschen 23 in die Einsteckschlitze 17 des ausgewählten Modulsteckplatzes 12.1 bis 12.4 einzustecken und dabei die beiden komplementär ausgebildeten Kontaktplatten 25, 18 aneinander elektrisch verbindend zur Anlage zu bringen.

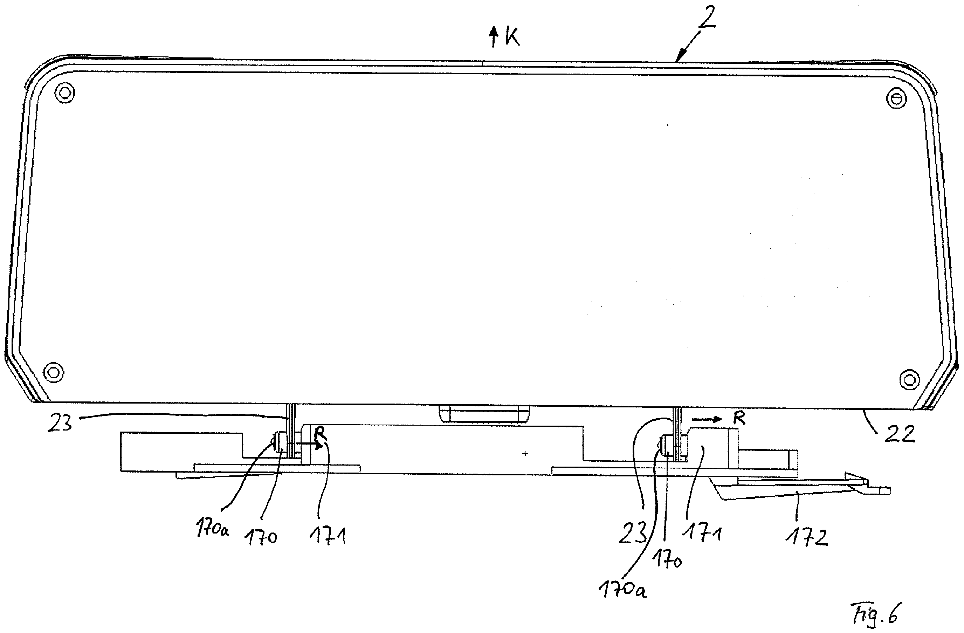

Sobald die Laschen 23 in die Einsteckschlitze 17 eines Modulsteckplatzes 12.1 bis 12.4 eingesteckt sind, greifen entgegen Pfeil R federbelastete Verriegelungsbolzen 170, die in der Figur 6 dargestellt sind, wobei zur besseren Übersichtlichkeit das Gehäuse des Basisgeräts 1 nicht eingezeichnet ist, in zwei der Bohrungen 24 innerhalb der Laschen 23 ein und verriegeln das Funktionsmodul in der in den Figuren 1 bzw. 2 dargestellten Position am ausgewählten Modulsteckplatz. Hierbei kann es sich um die unteren oder oberen Bohrungen 24 jeder Lasche 23 handeln. Eine um die Kontaktplatte 18 herum angeordnete Dichtung 19 sorgt dabei für eine den Anforderungen entsprechende Abdichtung der elektrischen Kontakte zwischen den Kontaktplatten 18 und 25.

Die Befestigung erfolgt im dargestellten Ausführungsbeispiel durch Anbringen des Funktionsmoduls 2 am gewünschten Modulsteckplatz 12.1, 12.2, 12.3 oder 12.4 in einer senkrecht zur Rückseite 11 des Basisgeräts 1 verlaufenden Andockrichtung D, die durch die Gestaltung der Kontaktplatten 18 und 25 sowie die Erstreckung der Laschen 23 und Einsteckschlitze 17 vorgegeben ist. Insoweit erfolgt das Andocken und mechanische sowie elektrische Verbinden der Funktionsmodule 2 am gewünschten Modulsteckplatz 12.1,12.2,12.3, 12.4 senkrecht zur Andockfläche A.

Darüber hinaus lassen sich die einzelnen Funktionsmodule 2 in entgegengesetzter Richtung entgegen Pfeil D senkrecht zur Andockfläche A in der nachfolgend noch näher beschriebenen Weise vom jeweiligen Modulsteckplatz 12.1, 12.2, 12.3, 12.4 auch wieder abdocken und entfernen.

Es ist somit offensichtlich, dass das Basisgerät 1 modular mit entsprechenden Funktionsmodulen 2 unterschiedlicher gewünschter Funktionalität ausgerüstet werden kann. Sobald ein Funktionsmodul 2 in einer der vorangehend beschriebenen Weise am Basisgerät 1 angedockt worden ist, kommunizieren die Datenverarbeitungseinrichtungen innerhalb des Basisgeräts und des Funktionsmoduls miteinander über die Kontakte in den Kontaktplatten 25, 18 und es erfolgt eine Anmeldung des Funktionsmoduls 2 am Basisgerät 1 und eine nachfolgende Nutzung der im Funktionsmodul 2 verbauten Funktionalität über das Basisgerät 1 im Sinne eines modularen Intensivtherapiegeräts. Die Stromversorgung jedes Funktionsmoduls 2 wird dabei vom Basisgerät 1 sichergestellt, und zwar entweder über einen eingebauten Akku bzw. Batterien im Mobilbetrieb, beispielsweise im Rahmen des Patiententransports oder im Rettungsdienst oder aber über eine angeschlossene Netzversorgung, wozu das Basisgerät über eine entsprechende Anschlussbuchse 15 verfügt.

Es ist auch möglich, mehrere Basisgeräte 1 nach Art einer Master-Slave-Anordnung oder "Ad hoc" zusammenzuschalten, um auf diese Weise die Anzahl an verfügbaren Modulsteckplätzen zu vervielfachen. Bei einer Master-Slave Anordnung übernimmt ein Basisgerät "Master" die Steuerung aller weiteren Module an den "Slave"-Basisgeräten, während bei einer "Ad hoc"-Zusammenschaltung alle Basisgeräte 1 Steuerungsfunktionen für ihre jeweiligen Funktionsmodule 2 beibehalten, aber ohne gegenseitige Ansteuerung miteinander kommunizieren können.

Wenn ein solchermaßen ausgebildetes und mit mehreren Funktionsmodulen 2 ausgebildetes modulares Intensivtherapiegerät z.B. im Bereich einer Intensivstation an einem Patientenbett positioniert wird, ist nicht von vorneherein festgelegt, ob die in der Darstellung gemäß Figur 1 von der Vorderseite 10 her betrachtet auf der rechten Seite des Funktionsmoduls 2 positionierten Anschlüsse des Anschlussfeldes 21 zum Patienten weisen und damit eine einfache und auf kurzem Weg erfolgende Konnektierung ermöglichen oder aber ob es vorteilhafter wäre, das Anschlussfeld 21 auf der anderen, hier linken Seite zu positionieren.

Wie aus der vergleichenden Betrachtung der Figur 1 mit der Figur 2 ersichtlich, ist es beim dargestellten modularen Intensivtherapiegerät ohne weiteres möglich, sämtliche Funktionsmodule auch in einer um 180° gewendeten Anordnung am Basisgerät 1 anzudocken und mit diesem zu verbinden, da keine Vorzugsrichtung vorherrscht und, wie aus den Figuren 3 und 5 ersichtlich, eine symmetrische Anordnung der Einsteckschlitze 17 sowie der Laschen 23 zur jeweiligen Mittelachse M der Modulsteckplätze 12.1 bis 12.4 bzw. MF des Moduls 2 gewählt ist und auch die Kontaktplatten 18, 25, die gemeinsam die elektrische Verbindungseinrichtungen bilden, symmetrisch auf diesen Mittelachsen M bzw. MF angeordnet sind. Darüber hinaus handelt es sich bei den Kontaktplatten 18, 25 um dual orientierte Kontaktplatten, deren Kontakte jeweils in einer um 180° versetzten Anordnung dupliziert sind, so dass gemäß der Darstellung in Figur 1 im Vergleich zu Figur 2 sowohl die aus Figur 1 ersichtliche Anordnung als auch die um 180° gewendete Orientierung gemäß Figur 2 vorgesehen sein kann, in welcher die Funktionsmodule 2 gleichermaßen am Basisgerät 1 befestigt werden können.

Nebenbei bemerkt ist zwar in der Darstellung der Figuren 1 und 2 lediglich ein Funktionsmodul 2 in der an einem Modulsteckplatz 12.2 angedockten Anordnung ersichtlich, jedoch versteht es sich, dass die weiteren Modulsteckplätze 12.1, 12.3 und 12.4 ebenfalls in dieser Weise mit Funktionsmodulen 2 bestückt sein können.

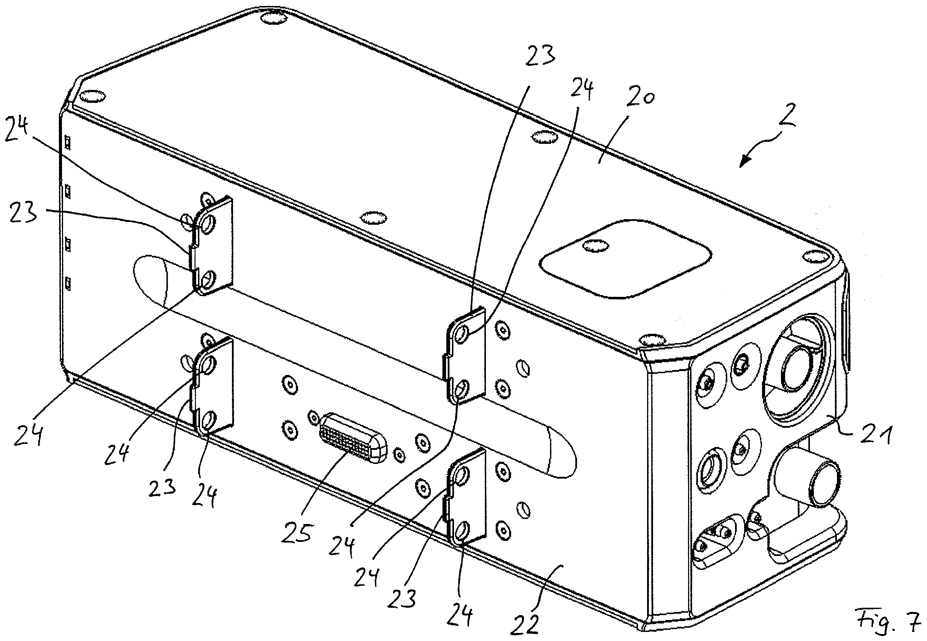

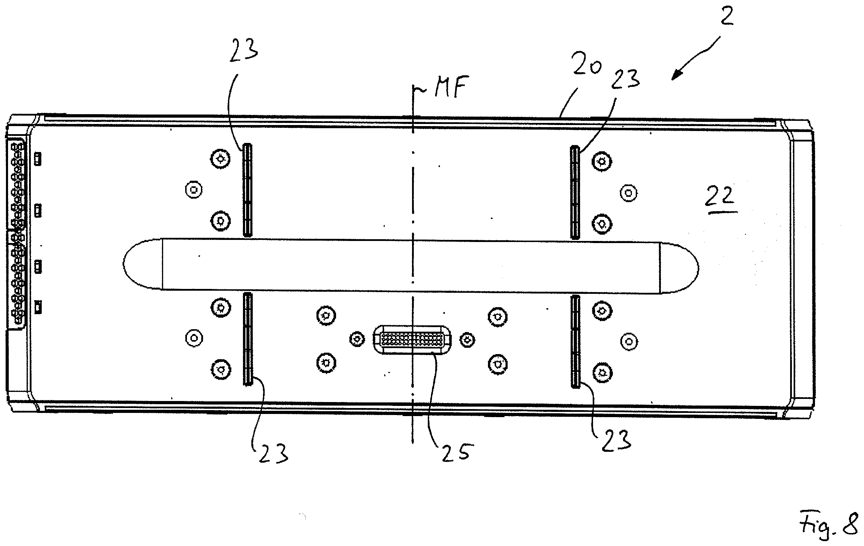

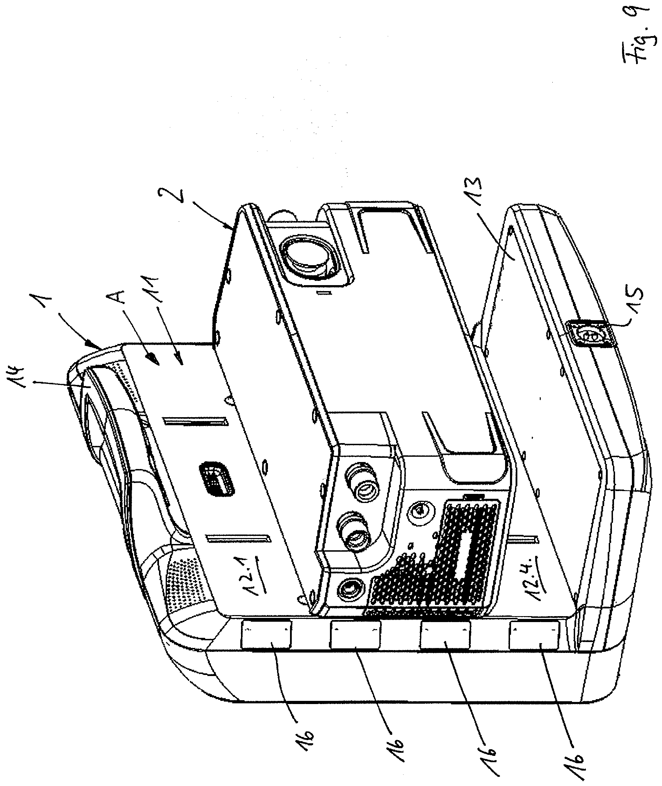

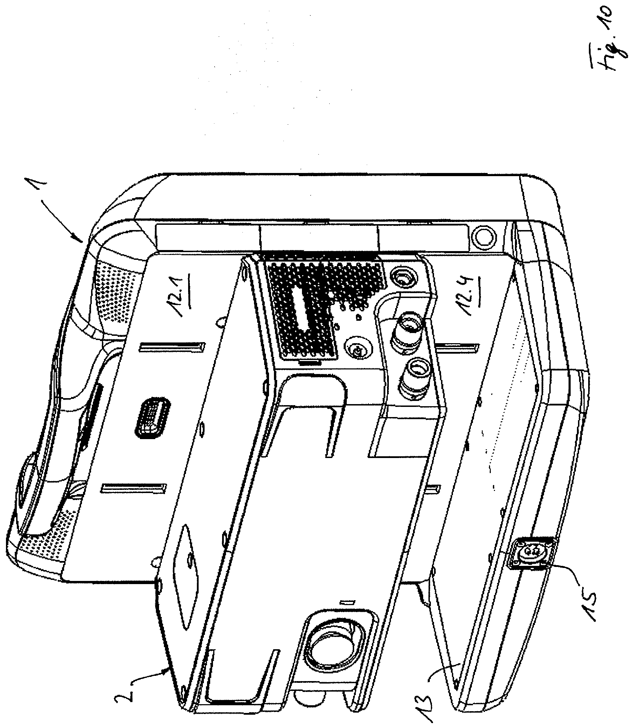

Darüber hinaus ist es auch möglich, Funktionsmodule 2 vorzusehen, die mehrere Modulsteckplätze belegend an dem Basisgerät 1 lösbar verriegelbar und mit diesen elektrisch verbindbar sind, etwa wenn die im Funktionsmodul 2 aufgenommene Funktionalität größeren Raumbedarf hat. Ein solches, zwei Modulsteckplätze belegendes Funktionsmodul 2 ist aus den Figuren 7 und 8 sowie in angedockter Position in zwei unterschiedlichen Orientierungen aus den Figuren 9 und 10 ersichtlich. Entsprechend den vorangehenden Erläuterungen umfasst ein solches über zwei Modulsteckplätze sich erstreckendes Funktionsmodul 2 insgesamt vier vorstehende Laschen 23 mit entsprechenden Durchgangsbohrungen 24, jedoch lediglich eine Kontaktplatte 25 und ist in den Figuren 9 und 10 beispielhaft an den Modulsteckplätzen 12.2 sowie 12.3 angedockt. Bei einem solchen größeren Modul 2 kann es sich beispielsweise um ein Beatmungsmodul handeln. Selbstverständlich sind auch Module vorstellbar, die über drei oder vier derartige Modulsteckplätze sich erstrecken und diese bei Konnektierung belegen.

Die vorangehend erläuterte Befestigung der Funktionsmodule 2 an den Modulsteckplätzen 12.1 bis 12.4 des Basisgeräts 1 ist selbstverständlich lösbar ausgebildet, d.h. im Falle eines Defekts eines einzelnen Funktionsmoduls 2 oder aber bei sich verändernden Therapieanforderungen können einzelne Funktionsmodule 2 aus ihrer am Basisgerät 1 angedockten Position an einem der Modulsteckplätze 12.1 bis 12.4 entfernt werden.

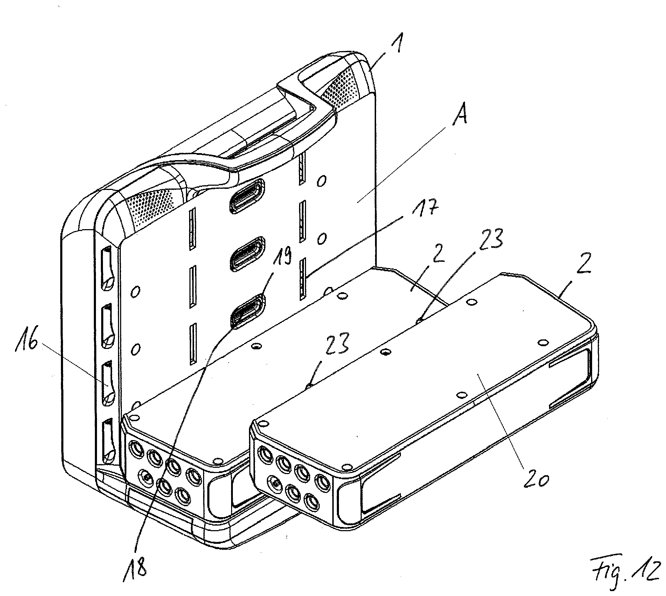

Zu diesem Zweck umfasst das Basisgerät 1 an einer seiner Seiten jedem Modulsteckplatz 12.1 bis 12.4 zugeordnet ein Freigabeorgan 16, bei dem es sich beispielsweise um einen Hebel oder eine Taste handeln kann. Die Ausführungsform gemäß Figur 1 zeigt Freigabeorgane 16 in Form von Drucktasten, hingegen zeigt die vergleichbare Ausführungsform gemäß Figur 12 Freigabeorgane 16 in Form von Hebeln. Durch Betätigung dieses Freigabeorgans 16 werden die aus der Darstellung gemäß Figur 6 ersichtlichen Antriebe 171 über ein entsprechendes Betätigungsorgan 172 angesprochen und bewirken ein Herausziehen der durch die Bohrungen 24 der Laschen 23 durchgreifenden und die Funktionsmodule 2 am Basisgerät 1 verriegelnden federbelasteten Verriegelungsbolzen 170 in Pfeilrichtung R, so dass die Funktionsmodule 2 freigegeben und vom jeweiligen Modulsteckplatz 12.1 bis 12.4 abgezogen werden können, indem die Laschen 23 aus den Einsteckschlitzen 17 herausgezogen werden.

Mit Vorteil erfolgt dieser Rückzug der Verriegelungsbolzen 170 aus den Bohrungen 24 in Pfeilrichtung R beim Betätigen des Freigabeorgans 16 nur soweit, dass das vordere Ende der Verriegelungsbolzen 170, welches leicht angefast ist, und mit Bezugszeichen 170a in Figur 6 versehen ist, nach wie vor innerhalb der Bohrungen 24 verbleibt, so dass das solchermaßen freigegebene Funktionsmodul 2 nach wie vor durch den entgegen Pfeilrichtung R federbelasteten Verriegelungsbolzen 170 am Basisteil 1 gesichert ist, es jedoch möglich ist, durch Aufbringen eines entsprechenden vorbestimmbaren Kraftaufwandes, etwa durch Ergreifen und Abziehen des Funktionsmoduls 2 in Pfeilrichtung K die Verriegelungsbolzen 170 entgegen der Federvorspannung aus den Bohrungen 24 vollständig herauszudrücken und das Funktionsmodul 2 abzunehmen.

Darüber hinaus kann vorgesehen sein, dass bei Betätigung des Freigabeorgans 16 ein Sensor ausgelöst wird, der zunächst ein Herunterfahren der Software des freizugebenden Funktionsmoduls 2 initiiert, bevor die Aktuatoren 171 angesprochen werden und einen Rückzug der Verriegelungsbolzen 170 in der soeben beschriebenen Weise gemäß Pfeil R auslösen. Hierfür kann beispielsweise ein entsprechender Hallsensor an den Freigabeorganen 16 vorgesehen sein, der mit einer entsprechenden Steuerungseinrichtung verbunden ist, die die zeitverzögerte Aktivierung der Antriebe 171 bewirkt.

Diese Steuerungseinrichtung kann außerdem mit einem Sensor zur Erfassung der in die Einsteckschlitze 17 eingesteckten Laschen 23 verbunden sein, so dass bei nicht erfolgtem Herausziehen der Laschen in Pfeilrichtung K gemäß Figur 6 binnen eines vorgebbaren Zeitintervalls die Antriebe 171 von der Steuerungseinrichtung wieder deaktivierbar sind und die Verriegelungsbolzen 170 entgegen Pfeilrichtung R infolge ihrer Federvorspannung erneut die Laschen 23 verriegeln. Auf diese Weise wird einem versehentlichen Lösen der Funktionsmodule durch unbeabsichtigtes Auslösen des Freigabeorgans 16 vorgebeugt.

Durch die vorangehend dargestellte Ausgestaltung ist die modulare Ausrüstung eines Basisgeräts mit mindestens einem Funktionsmodul zu einem modularen Intensivtherapiegerät möglich, wobei die einzelnen Funktionsmodule keine Vorzugsrichtung aufweisen, sondern in zwei um 180° versetzt zueinander ausgebildeten Orientierungen je nach Anwendungsfall am Basisgerät 1 befestigbar sind. Die bewirkte Verriegelung und Konnektierung am Basisgerät 1 ist hierbei fehlersicher und mechanisch robust ausgeführt, so dass auch die einschlägigen Rütteltestanforderungen für den mobilen Einsatz eines solchen modularen Intensivtherapiegeräts beispielsweise in Rettungsfahrzeugen oder Rettungshubschraubern erreicht werden.

Zur Sicherstellung einer ausreichenden elektrischen Verbindung zwischen dem Basisgerät 1 und den Funktionsmodulen 2 auch unter dem Gesichtspunkt der Rütteltests kann von daher auch vorgesehen sein, die Kontaktplatten 18 im Basisgerät 1 und/oder die Kontaktplatten 25 der Funktionsmodule 2 federbelastet in Richtung auf die jeweils andere Kontaktplatte anzuordnen, beispielsweise können die Kontaktplatten 18 bzw. 25 schwimmend gelagert sein oder aber die Einzelkontakte in den Kontaktplatten 18, 25 sind mittels Federn in Richtung auf die jeweils andere Kontaktplatte vorgespannt.

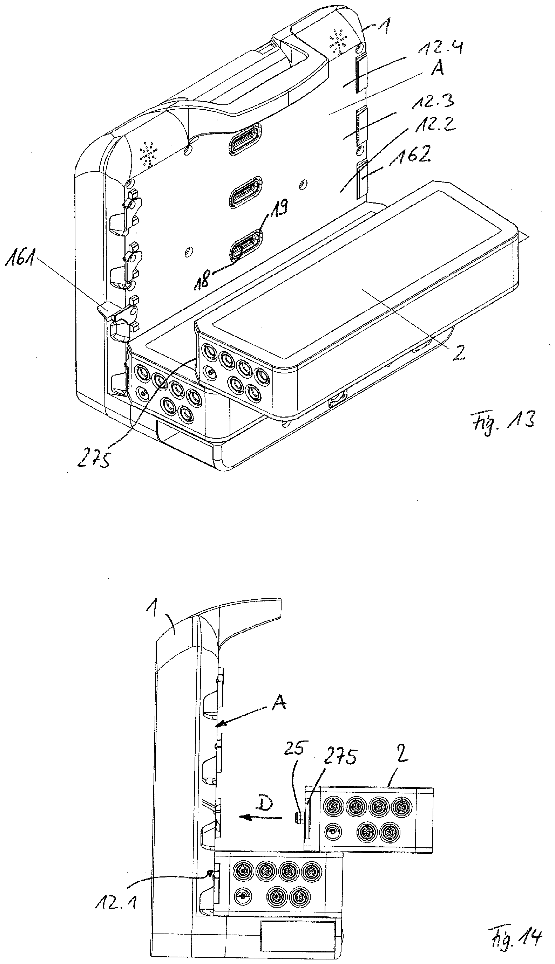

Aus den Figuren 13 und 14 ist eine weitere Ausführungsform des erfindungsgemäßen modularen Intensivtherapie Geräts ersichtlich, welches sich von den vorangehend erläuterten Ausführungsformen lediglich durch die Ausgestaltung der mechanischen Verbindungselemente für die mechanische Befestigung der einzelnen Funktionsmodule 2 an den ausgewählten Modulsteckplätzen 12.1,12.2,12.3 und 12.4 unterscheidet.

So ist dem dargestellten Ausführungsbeispiel die Verriegelungseinrichtung von an der Andockfläche A im Bereich jedes Modulsteckplatzes 12.1, 12.2, 12.3, 12.4 von vorstehenden Haken 162 und schwenkbaren Verriegelungshebeln 161 gebildet, die in entsprechende schlitzförmige Ausnehmungen 275 an den Schmalseiten der Funktionsmodule 2 zum Zwecke der mechanischen Befestigung eingreifen. Dabei sind vorstehende Haken 162 in der Darstellung gemäß Figur 13 am rechten Rand der Andockfläche A übereinander entsprechend der Anordnung der Modulsteckplätze 12.1,12.2,12.3,12.4 vorgesehen, während am gegenüberliegenden linken Rand der Andockfläche A entsprechend jeweils ein um 90° schwenkbarer Verriegelungshebel 161 als Freigabeorgan vorgesehen ist.

Auch bei dieser Ausführungsform gemäß Figuren 13 und 14 wird somit jedes einzelne Funktionsmodul 2 gemäß Pfeil D senkrecht zur Andockfläche A angedockt bzw. entgegen Pfeil D abgedockt. Dazu wird zunächst die schlitzförmige Ausnehmung 275 mit dem Haken 162 verhakt, anschließend wird das Funktionsmodul 2 so über der Andockfläche A eingedreht, dass die elektrische Verbindung zwischen den Kontaktplatten 18, 25 hergestellt wird und schließlich der am Modulsteckplatz 12.2 geöffnet dargestellte Verriegelungshebel 161 in eine geschlossene Stellung geschwenkt wird, die beispielsweise am Modulsteckplatz 12.3 zu sehen ist und in der der Verriegelungshebel 161 in die entsprechende Ausnehmung 275 am Funktionsmodul 2 zu dessen mechanischer Verriegelung eingreift.

Das Abdocken des Funktionsmoduls 2 erfolgt in entsprechend umgekehrter Reihenfolge und Vorgehensweise.

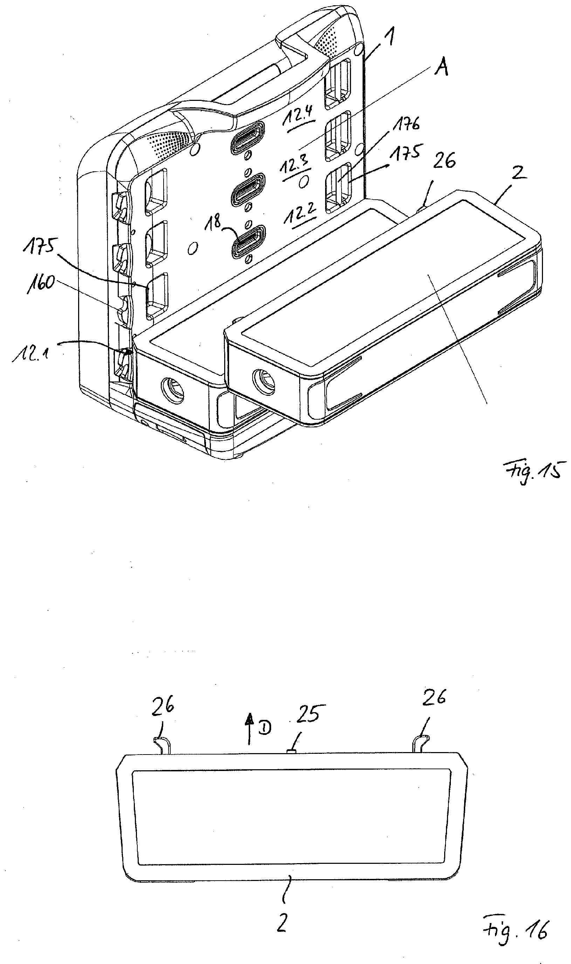

In den Darstellungen der Figuren 15 und 16 ist eine demgegenüber nochmals abgewandelte Ausführungsform der Erfindung dargestellt, bei der zur Ausbildung der Verriegelungseinrichtung vorstehende Haken 26 auf der der Andockfläche A zugewandten Seite des Funktionsmoduls 2 beidseits vorstehen.

Hierzu korrespondierend weist die Andockfläche A des Basisgeräts 1 im Bereich jedes Modulsteckplatzes 12.1,12.2,12.3,12.4 spiegelbildlich zur Kontaktplatte 18 Einstecköffnungen 175 auf, wobei die in der Darstellung gemäß Figur 15 am rechten Rand der Andockfläche A angeordneten Einstecköffnungen 175 jeweils einen vertikalen Steg 176 aufweisen, welchen der zugeordnete Haken 26 des Funktionsmoduls 2 in angedocktem Zustand hintergreift. Auf der gegenüberliegenden linken Seite der Andockfläche A sind Verriegelungsscheiben 160 angeordnet, die bei Drehung um ihre horizontal verlaufende Drehachse entweder die Einstecköffnung 175 freigegeben, um das Einführen eines Hakens 26 zu ermöglichen, oder in die Einstecköffnung 175 eingreifen, um einen eingeführten Haken 26 verriegelnd zu hintergreifen.

Auch diese Ausführungsform ermöglicht somit das An- und Abdocken der Funktionsmodule 2 an bzw. von den Modulsteckplätzen 12.1,12.2,12.3 und 12.4 in oder entgegen Pfeilrichtung D senkrecht zur Andockfläche A.

Darüber hinaus ist es auch bei den Ausführungsbeispielen gemäß Figuren 13 bis 16 möglich, die einzelnen Funktionsmodule 2 auch in einer um 180° gedrehten Orientierung am Basisgerät 1 zu befestigen.

## Patentansprüche

1. Modulares Intensivtherapiegerät, umfassend ein Basisgerät (1) mit mindestens einem in einer Andockfläche (A) ausgebildeten Modulsteckplatz (12.1, 12.2, 12.3, 12.4) sowie mindestens ein Funktionsmodul (2), welches an dem mindestens einen Modulsteckplatz (12.1, 12.2, 12.3, 12.4) an dem Basisgerät (1) andockbar und lösbar verriegelbar und elektrisch mit diesem verbindbar ist, wobei jeder Modulsteckplatz (12.1, 12.2, 12.3, 12.4) und jedes Funktionsmodul (2) mit zueinander komplementär ausgebildeten lösbaren Verriegelungseinrichtungen und elektrischen Verbindungseinrichtungen ausgebildet sind, wobei die Verriegelungseinrichtungen und die elektrischen Verbindungseinrichtungen im Bereich der Andockfläche (A) ausgebildet und so angeordnet sind, dass das mindestens eine Funktionsmodul (2) senkrecht zur Andockfläche (A) an dem mindestens einen Modulsteckplatz (12.1, 12.2, 12.3, 12.4) andockbar und lösbar verriegelbar ist, **dadurch gekennzeichnet, dass** die Verriegelungseinrichtungen von an den Funktionsmodulen (2) vorstehenden Laschen (23) mit diese durchsetzenden Bohrungen (24) und zugehörigen Einsteckschlitzen (17) in der Andockfläche (A) und in die Bohrungen (24) lösbar eingreifenden Verriegelungsbolzen (170) im Bereich jedes Modulsteckplatzes (12.1, 12.2, 12.3, 12.4) des Basisgeräts (1) gebildet sind und die Verriegelungsbolzen (170) federbelastet im Basisgerät (1) gehaltert sind und beim Einstecken der Laschen (23) in die Einsteckschlitze (17) in die Bohrungen (24) eingreifen und die Laschen verriegeln und mittels zugeordneter Antriebe (171) über am Basisgerät (1) angeordnete Freigabeorgane (16) aus den Bohrungen (24) teilweise herausziehbar sind, so dass die Laschen (23) unter Aufbringung einer vorbestimmten Zugkraft aus den Einsteckschlitzen (17) unter vollständigem Herausgleiten der Verriegelungsbolzen (170) aus den Bohrungen (24) entgegen der Federlast herausziehbar sind.

2. Intensivtherapiegerät nach Anspruch 1, **dadurch gekennzeichnet, dass** die Freigabeorgane (16) einen Sensor zur Erfassung einer Betätigung des jeweiligen Freigabeorgans (16) aufweisen und eine mit dem Sensor verbundene Steuerungseinrichtung vorgesehen ist, mittels derer bei vom Sensor erfasster Betätigung des Freigabeorgans (16) die Antriebe (171) zeitverzögert aktivierbar sind, um die Verriegelungsbolzen (170) aus den Bohrungen (24) herauszuziehen, wobei die Steuerungseinrichtung außerdem mit einem Sensor zur Erfassung der in die Einsteckschlitze (17) eingesteckten Laschen (23) verbunden ist, so dass bei nicht erfolgtem Herausziehen der Laschen (23) binnen eines vorgebbaren Zeitintervalls die Antriebe (171) von der Steuerungseinrichtung deaktivierbar sind und die Verriegelungsbolzen (170) erneut die Laschen (23) verriegeln.

3. Intensivtherapiegerät nach einem der Ansprüche 1 oder 2, **dadurch gekennzeichnet, dass** die Verriegelungseinrichtungen und die elektrischen Verbindungseinrichtungen des mindestens einen Funktionsmoduls (2) in zwei um 180 Grad zueinander versetzten Orientierungen an dem mindestens einen Modulsteckplatz (12.1, 12.2, 12.3, 12.4) verriegelbar und elektrisch mit diesem verbindbar sind.

4. Intensivtherapiegerät nach einem der Ansprüche 1 bis 3, **dadurch gekennzeichnet, dass** die Verriegelungseinrichtungen und die elektrischen Verbindungseinrichtungen symmetrisch zu einer durch den Modulsteckplatz (12.1, 12.2, 12.3, 12.4) verlaufenden Mittelachse (M) angeordnet sind.

5. Intensivtherapiegerät nach einem der Ansprüche 1 bis 4, **dadurch gekennzeichnet, dass** mindestens ein Funktionsmodul (2) vorgesehen ist, welches mehrere Modulsteckplätze (12.1, 12.2, 12.3, 12.4) belegend an dem Basisgerät (1) lösbar verriegelbar und elektrisch mit diesem verbindbar ist.

6. Intensivtherapiegerät nach einem der Ansprüche 1 bis 5, **dadurch gekennzeichnet, dass** die elektrischen Verbindungseinrichtungen der Funktionsmodule (2) und der Modulsteckplätze (12.1, 12.2, 12.3, 12.4) des Basisgeräts (1) von Kontaktplatten (18, 25) gebildet sind, die jeweils eine Vielzahl von Kontakten aufweisen, die in einer um 180 Grad versetzten Anordnung dupliziert sind und bei an einem Modulsteckplatz (12.1, 12.2, 12.3, 12.4) verriegeltem Funktionsmodul (2) aneinander zur Anlage kommen.

7. Intensivtherapiegerät nach Anspruch 6, **dadurch gekennzeichnet, dass** die Kontakte mindestens einer Kontaktplatte (18, 25) der elektrischen Verbindungseinrichtung federbelastet in Richtung der jeweils anderen Kontaktplatte (25, 18) angeordnet sind.

## Claims

1. Modular intensive therapy device, comprising a base unit (1) with at least one module slot (12.1, 12.2, 12.3, 12.4) formed in a docking surface (A) and at least one function module (2) which is connected to the at least one module slot (12.1, 12.2, 12.3, 12.4) on the base unit (1) and can be docked and releasably lockable and electrically connected thereto, wherein each module slot (12.1, 12.2, 12.3, 12.4) and each functional module (2) are formed with mutually complementary formed releasable locking means and electrical connection means, wherein the locking means and the electrical connecting means in the region of the docking surface (A) are formed and arranged so that the at least one function module (2) perpendicular to the docking surface (A) at the at least one module slot (12.1, 12.2, 12.3, 12.4) is dockable and releasably lockable, **characterized in that** the locking means are formed on the projecting tabs (23) of the functional modules (2) with these passing through holes (24) and associated insertion slots (17) in the docking surface (A), and in the holes (24) releasably engaging locking pins (170) in the region of each module slot (12.1, 12.2, 12.3, 12.4) of the base unit (1), and the locking pins (170) are held spring-loaded in the base unit (1), and when inserting the tabs (23) in the insertion slots (17) engage in the holes (24) and lock the tabs, and by means of associated actuators (171) via on the base unit (1) arranged release means (16) can be partially withdrawn from the holes (24), so that the tabs (23) can be pulled out while applying a predetermined tensile force from the insertion slots (17) with complete sliding out of the locking pins (170) from the holes (24) against the spring load.

2. Intensive therapy device according to claim 1, **characterized in that** the release means (16) have a sensor for detecting an actuation of the respective release mean (16) and a control device connected to the sensor is provided, by means of which when the sensor detects actuation of the release means (16) actuators (171) are activated with a time delay in order to pull out the locking pins (170) from the holes (24), wherein the control device is also connected to a sensor for detecting tabs (23) inserted into the insertion slots (17), so that on unsuccessful pulling out of the tabs (23) within a predeterminable time interval, the actuators (171) can be deactivated by the control device and the locking pins (170) lock the tabs (23) again.

3. Intensive therapy device according to one of claims 1 or 2, **characterized in that** the locking means and the electrical connection means of the at least one functional module (2) in two mutually offset by 180 degrees orientations on the at least one module slot (12.1, 12.2, 12.3, 12.4) are lockable and electrically connected to this.

4. Intensive therapy device according to one of claims 1 to 3, **characterized in that** the locking means and the electrical connection means are arranged symmetrically to a through the module slot (12.1, 12.2, 12.3, 12.4) extending central axis (M).

5. Intensive therapy device according to one of claims 1 to 4, **characterized in that** at least one function module (2) is provided, to which a plurality of module slots (12.1, 12.2, 12.3, 12.4) occupying the base unit (1) are releasably lockable and electrically connected.

6. Intensive therapy device according to one of claims 1 to 5, **characterized in that** the electrical connection means of the function modules (2) and the module slots (12.1, 12.2, 12.3, 12.4) of the base unit (1) are formed of contact plates (18, 25), which respectively have a plurality of contacts, which are duplicated in a 180-degree offset arrangement, and come in abutment to each other at a function module (2) locked to a module slot (12.1, 12.2, 12.3, 12.4).

7. Intensive therapy device according to claim 6, **characterized in that** the contacts of at least one contact plate (18, 25) of the electrical connection device are spring-loaded in the direction of the respective other contact plate (25, 18).

## Revendications

1. Appareil de soins intensifs modulaire, comprenant un appareil de base (1) avec au moins un emplacement d'enfichage de module (12.1, 12.2, 12.3, 12.4) réalisé dans une surface d'arrimage (A) ainsi qu'au moins un module fonctionnel (2) qui peut être arrimé et verrouillé de manière amovible au niveau de l'au moins un emplacement d'enfichage de module (12.1, 12.2, 12.3, 12.4) à l'appareil de base (1) et peut être relié électriquement à celui-ci, dans lequel chaque emplacement d'enfichage de module (12.1, 12.2, 12.3, 12.4) et chaque module fonctionnel (2) sont réalisés avec des dispositifs de verrouillage amovibles et des dispositifs de liaison électriques réalisés complémentaires les uns des autres, dans lequel les dispositifs de verrouillage et les dispositifs de liaison électriques sont réalisés et agencés dans la zone de la surface d'arrimage (A) de telle sorte que l'au moins un module fonctionnel (2) peut être arrimé et verrouillé de manière amovible perpendiculairement à la surface d'arrimage (A) au niveau de l'au moins un emplacement d'enfichage de module (12.1, 12.2, 12.3, 12.4), **caractérisé en ce que** les dispositifs de verrouillage sont formés par des éclisses (23) dépassant au niveau des modules fonctionnels (2) avec des trous (24) traversant celles-ci et des fentes d'insertion associées (17) dans la surface d'arrimage (A) et des axes de verrouillage (170) pénétrant de manière amovible dans les trous (24) dans la zone de chaque emplacement d'enfichage de module (12.1, 12.2, 12.3, 12.4) de l'appareil de base (1) et les axes de verrouillage (170) sont maintenus par une contrainte de ressort dans l'appareil de base (1) et, lors de l'insertion des éclisses (23) dans les fentes d'insertion (17), pénètrent dans les trous (24) et verrouillent les éclisses et peuvent être retirés partiellement des trous (24) au moyen de dispositifs d'entraînement associés (171) par l'intermédiaire d'organes de dégagement (16) agencés au niveau de l'appareil de base (1) de telle sorte que, en appliquant une force de traction prédéterminée, les éclisses (23) peuvent être retirées des fentes d'insertion (17) en faisant complètement glisser les axes de verrouillage (170) hors des trous (24) contre la force de ressort.

2. Appareil de soins intensifs selon la revendication 1, **caractérisé en ce que** les organes de dégagement (16) comportent un capteur destiné à la détection de l'actionnement de l'organe de dégagement (16) respectif et un dispositif de commande relié au capteur est prévu, dispositif de commande au moyen duquel, lors de l'actionnement de l'organe de dégagement (16) détecté par le capteur, les dispositifs d'entraînement (171) peuvent être activés avec retard pour retirer les axes de verrouillage (170) hors des trous (24), lequel dispositif de commande est relié en outre à un capteur destiné à la détection des éclisses (23) insérées dans les fentes d'insertion (17) de telle sorte que, lorsque le retrait des éclisses (23) n'est pas effectué dans les limites d'un intervalle de temps pouvant être prescrit, les dispositifs d'entraînement (171) peuvent être désactivés par le dispositif de commande et les axes de verrouillage (170) verrouillent à nouveau les éclisses (23).

3. Appareil de soins intensifs selon l'une quelconque des revendications 1 ou 2, **caractérisé en ce que** les dispositifs de verrouillage et les dispositifs de liaison électriques de l'au moins un module fonctionnel (2) peuvent être verrouillés dans deux orientations décalées de 180 degrés l'une par rapport à l'autre au niveau de l'au moins un emplacement d'enfichage de module (12.1, 12.2, 12.3, 12.4) et peuvent être reliés électriquement à celui-ci.

4. Appareil de soins intensifs selon l'une quelconque des revendications 1 à 3, **caractérisé en ce que** les dispositifs de verrouillage et les dispositifs de liaison électriques sont agencés symétriquement par rapport à un axe médian (M) passant par l'emplacement d'enfichage de module (12.1, 12.2, 12.3, 12.4).

5. Appareil de soins intensifs selon l'une quelconque des revendications 1 à 4, **caractérisé en ce qu'**il est prévu au moins un module fonctionnel (2) qui, en occupant plusieurs emplacements d'enfichage de module (12.1, 12.2, 12.3, 12.4), peut être verrouillé de manière amovible au niveau de l'appareil de base (1) et peut être relié électriquement à celui-ci.

6. Appareil de soins intensifs selon l'une quelconque des revendications 1 à 5, **caractérisé en ce que** les dispositifs de liaison électriques des modules fonctionnels (2) et des emplacements d'enfichage de module (12.1, 12.2, 12.3, 12.4) de l'appareil de base (1) sont formés par des plaques de contact (18, 25) qui comportent chacune plusieurs contacts qui sont dupliqués dans un agencement décalé de 180 degrés et qui viennent en appui les uns contre les autres lorsqu'un module fonctionnel (2) est verrouillé au niveau d'un emplacement d'enfichage de module (12.1, 12.2, 12.3, 12.4).

7. Appareil de soins intensifs selon la revendication 6, **caractérisé en ce que** les contacts d'au moins une plaque de contact (18, 25) du dispositif de liaison électrique sont agencés sous la contrainte d'un ressort en direction de l'autre plaque de contact respective (25, 18).
